# EUROPEAN PATENT SPECIFICATION

(11) **EP 1 062 202 B1**
(45) Date of publication and mention of the grant of the patent: **15.01.2003**
(21) Application number: 98909620.1
(22) Date of filing: 12.03.1998
(51) Int. Cl.: C07C 259/06, C07D 333/24, A61K 31/22, A61K 31/38

(54) **CYTOSTATIC AGENTS**
CYTOSTATIKA
CYTOSTATIQUES

(43) Date of publication of application: 27.12.2000
(73) Proprietor: BRITISH BIOTECH PHARMACEUTICALS LIMITED, Cowley Oxford, OX4 5LY (GB)
(72) Inventor: PEARSON, Lindsey, Ann, Cowley, Oxford OX4 5LY (GB); AYSCOUGH, Andrew, Paul, Cowley, Oxford OX4 5LY (GB); HUXLEY, Philip, Cowley, Oxford OX4 5LY (GB); DRUMMOND, Alan, Hastings, Cowley, Oxford OX4 5LY (GB)
(74) Representative: Walls, Alan James
(86) International application number: GB9800754
(87) International publication number: WO99046241

(56) References cited:
- EP-A- 9 321 942
- WO-A-96/33166
- WO-A-98/11063
- GB-A- 2 268 934

## Description

The present invention relates to therapeutically active esters and thioesters, to processes for their preparation, to pharmaceutical compositions containing them, and to the use of such compounds in medicine. In particular, the compounds are inhibitors of the proliferation of a range of rapidly dividing tumour cells, for example melanoma and/or lymphoma cells.

### Background to the Invention

Our international patent application no. PCT/GB97/02398 describes and claims a method for inhibiting proliferation of tumour cells in mammals, comprising administering to the mammal suffering such proliferation an amount of a compound of general formula (I) or a pharmaceutically acceptable salt hydrate or solvate thereof sufficient to inhibit such proliferation: 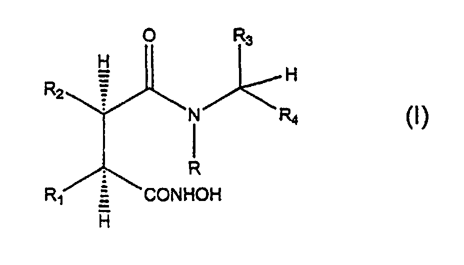 wherein
- R: is hydrogen or (C₁-C₆)alkyl;
- R₁: is hydrogen;
(C₁-C₆)alkyl;
(C₂-C₆)alkenyl;
phenyl or substituted phenyl;
phenyl (C₁-C₆)alkyl or substituted phenyl(C₁-C₆)alkyl;
phenyl (C₂-C₆)alkenyl or substituted phenyl(C₂-C₆)alkenyl
heterocyclyl or substituted heterocyclyl;
heterocyclyl(C₁-C₆)alkyl or substituted heterocyclyl(C₁-C₆)alkyl;
a group BSOₙA- wherein n is 0, 1 or 2 and B is hydrogen or a (C₁-C₆) alkyl, phenyl, substituted phenyl, heterocyclyl substituted heterocyclyl, (C₁-C₆)acyl, phenacyl or substituted phenacyl group, and A represents (C₁-C₆)alkylene;
hydroxy or (C₁-C₆)alkoxy;
amino, protected amino, acylamino, (C₁-C₆)alkylamino or di-(C₁-C₆)alkylamino;
mercapto or (C₁-C₆)alkylthio;
amino(C₁-C₆)alkyl, (C₁-C₆)alkylamino(C₁-C₆)alkyl, di(C₁-C₆)alkylamino(C₁-C₆)alkyl, hydroxy(C₁-C₆)alkyl, mercapto(C₁-C₆)alkyl or carboxy(C₁-C₆) alkyl wherein the amino-, hydroxy-, mercapto- or carboxyl-group are optionally protected or the carboxyl- group amidated;
lower alkyl substituted by carbamoyl, mono(lower alkyl)carbamoyl, di(lower alkyl)carbamoyl, di(lower alkyl)amino, or carboxy-lower alkanoylamino; or
a cycloalkyl, cycloalkenyl or non-aromatic heterocyclic ring containing up to 3 heteroatoms, any of which may be (i) substituted by one or more substituents selected from C₁-C₆ alkyl, C₂-C₆ alkenyl, halo, cyano (-CN), -CO₂H, -CO₂R, - CONH₂, -CONHR, -CON(R)₂, -OH, -OR, oxo-, -SH, -SR, -NHCOR, and - NHCO₂R wherein R is C₁-C₆ alkyl or benzyl and/or (ii) fused to a cycloalkyl or heterocyclic ring;
- R₂: is a C₁-C₁₂ alkyl,
C₂-C₁₂ alkenyl,
C₂-C₁₂ alkynyl,
phenyl(C₁-C₆ alkyl)-,
heteroaryl(C₁-C₆ alkyl)-,
phenyl(C₂-C₆ alkenyl)-,
heteroaryl(C₂-C₆ alkenyl)-,
phenyl(C₂-C₆ alkynyl)-,
heteroaryl(C₂-C₆ alkynyl)-,
cycloalkyl(C₁-C₆ alkyl)-,
cycloalkyl(C₂-C₆ alkenyl)-,
cycloalkyl(C₂-C₆ alkynyl)-,
cycloalkenyl(C₁-C₆ alkyl)-,
cycloalkenyl(C₂-C₆ alkenyl)-,
cycloalkenyl(C₂-C₆ alkynyl)-,
phenyl(C₁-C₆ alkyl)O(C₁-C₆ alkyl)-, or
heteroaryl(C₁-C₆ alkyl)O(C₁-C₆ alkyl)- group,
any one of which may be optionally substituted by
C₁-C₆ alkyl,
C₁-C₆ alkoxy,
halo,
cyano (-CN),
phenyl, or
phenyl substituted by
C₁-C₆ alkyl,
C₁-C₆ alkoxy,
halo, or
cyano (-CN);
- R₃: is the characterising group of a natural or non-natural α amino acid in which any functional groups may be protected; and
- R₄: is an ester or thioester group,
or a pharmaceutically acceptable salt, hydrate or solvate thereof.

### Brief Description of the invention

This invention relates to certain specific compounds which are inhibitors of the proliferation of tumour cells in mammals. The compounds in question are not specifically disclosed in PCT/GB97/02398, and have valuable pharmacological and pharmacokinetic properties as tumour cell inhibitors.

The use of matrix metallo proteases containing a hydroxamic group as inhibitors of tumour growth is further disclosed in WO-9321942.

### Detailed description of the invention

According to the present invention there is provided a compound selected from the group consisting of:
2(R or S)-[2R-(S-Hydroxy-hydroxycarbamoyl-methyl)-4-methyl-pentanoylamine]-2-phenyl-ethanoic acid cyclopentyl ester,
2(R or S)-(3S-Hydroxycarbamoyl-2R-isobutyl-hex-5-enoylamino)-2-phenylethanoic acid isopropyl ester,
2(R or S)-[2R-(S-Hydroxycarbamoyl-methoxy-methyl)-4-methyl-pentanoylamino]-3-phenylethanoic acid cyclopentyl ester,
2(R or S)-(3S-Hydroxycarbamoyl-2R-isobutyl-hex-5-enoylamino)-2-(4-methoxyphenyl)ethanoic acid cyclopentyl ester,
2(R or S)-(3S-Mydroxycarbamoyl-2R-isobutyl-hex-5-enoylamino)-2-(thien-2-yl)ethanoic acid cyclopentyl ester,
2(R or S)-(3S-Hydroxycarbamoyl-2R-isobutyl-hex-5-enoylamino)-2-(thien-3-yl)ethanoic acid cyclopentyl ester,
and pharmaceutically or veterinarily acceptable salts, hydrates or solvates thereof.

The 2-S diastereomers of the above compounds are preferred.

Salts of the compounds of the invention include physiologically acceptable acid addition salts for example hydrochlorides, hydrobromides, sulphates, methane sulphonates, p-toluenesulphonates, phosphates, acetates, citrates, succinates, lactates, tartrates, fumarates and maleates. Salts may also be formed with bases, for example sodium, potassium, magnesium, and calcium salts.

In another of its aspects, the invention comprises a method for inhibiting proliferation of tumour cells in mammals, comprising administering to the mammal suffering such proliferation an amount of a compound specified above or a pharmaceutically or veterinarily acceptable salt, hydrate or solvate thereof, sufficient to inhibit such proliferation.

In another aspect, the invention comprises the use of a compound compound specified above or a pharmaceutically or veterinarily acceptable salt, hydrate or solvate thereof, in the manufacture of a composition for inhibiting proliferation of tumour cells in mammals.

The compounds of the invention are useful in human or veterinary medicine since they are active as inhibitors of the proliferation of cancer cells. The utility of the invention therefore lies in the treatment of cancers, such as those caused by over-proliferation of lymphoma, leukemia, myeloma, adenocarcinoma, carcinoma, mesothelioma, teratocarcinoma, choriocarcinoma, small cell carcinoma, large cell carcinoma, melanoma, retinoblastoma, fibrosarcoma, leiomyosarcoma, glioblastoma or endothelioma cells. It will be understood that different compounds of the invention will have differing potencies as proliferation inhibitors depending on the the type of cancer being treated. The activity of any particular compound of the invention in inhibiting proliferation of any particular cell type may be routinely determined by standard methods, for example analagous to those described in the Biological Example herein.

In a further aspect of the invention there is provided a pharmaceutical or veterinary composition comprising a compound of the invention, together with a pharmaceutically or veterinarily acceptable excipient or carrier. One or more compounds of the invention may be present in the composition together with one or more excipient or carrier.

Orally administrable compositions may be in the form of tablets, capsules, powders, granules, lozenges, liquid or gel preparations, such as oral, topical, or sterile parenteral solutions or suspensions. Tablets and capsules for oral administration may be in unit dose presentation form, and may contain conventional excipients such as binding agents, for example syrup, acacia, gelatin, sorbitol, tragacanth, or polyvinyl-pyrrolidone; fillers for example lactose, sugar, maize-starch, calcium phosphate, sorbitol or glycine; tabletting lubricant, for example magnesium stearate, talc, polyethylene glycol or silica; disintegrants for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For topical application to the skin, the drug may be made up into a cream, lotion or ointment. Cream or ointment formulations which may be used for the drug are conventional formulations well known in the art, for example as described in standard textbooks of pharmaceutics such as the British Pharmacopoeia.

The active ingredient may also be administered parenterally in a sterile medium. Depending on the vehicle and concentration used, the drug can either be suspended or dissolved in the vehicle. Advantageously, adjuvants such as a local anaesthetic, preservative and buffering agents can be dissolved in the vehicle.

It will be understood that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The compounds of the invention (Examples 1-6) were prepared using procedures similar to those described in Examples 8, 16, 3, and 41 of our international patent application PCT/GB97/02398. Those Examples are reproduced below as Preparative Examples A-D respectively. Products obtained as mixtures of diastereoisomers were separated by reverse phase hplc. In the Examples the following abbreviations have been used:
DCM - Dichloromethane
DMF - N,N-Dimethylformamide
NMM - N-Methylmorpholine
TFA - Trifluoroacetic acid
HOBT- 1-Hydroxybenzotriazole

Column chromatography was performed with flash grade silica gel. ¹H-NMR and ¹³C-NMR were recorded on a Bruker AC 250E spectrometer at 250.1 and 62.9MHz respectively. CDCl₃ methanol-d₄ and dimethysulphoxide-d₆ (DMSO-d₆) were used as solvents and internal reference and spectra are reported as δ ppm from TMS.

### Preparative Example A

### 2S-[2R-(S-Hydroxy-hydroxycarbamoyl-methyl)-4-methyl-pentanoylamine]-3-phenyl-propionic acid isopropyl ester.

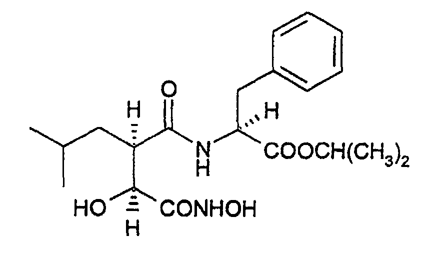

### (a) 2S-[2R-(2,2-Dimethyl-5-oxo-[1,3]-dioxolan-4S-yl)-4-methyl-pentanoylamino]-3-phenylpropionic acid isopropyl ester.

A solution of 2R-(2,2-dimethyl-5-oxo-[1,3-dioxolan-4S-yl)-4-methyl-pentanoic acid pentafluorophenyl ester (WO 95/19956) (2.87g, 7.3mmol) and L-phenylalanine isopropyl ester (1.5g, 7.3mmol) in DCM was allowed to stand at room temperature for 96 hours. The reaction mixture was diluted with DCM and washed with 1M aqueous sodium carbonate, 1M hydrochloric acid and brine before drying over magnesium sulphate, filtration and concentration under reduced pressure. The product was recrystallised from ethyl acetate/hexane to yield 2S-[2R-(2,2-dimethyl-5-oxo-[1,3]-dioxolan-4S-yl)-4-methyl-pentanoylamino]-3-phenylpropionic acid isopropyl ester as fine white needles (810mg, 29%).

### (b) 2S-[2R-(S-Hydroxy-hydroxycarbamoyl-methyl)-4-methyl-pentanoylamine]-3-phenyl-propionic acid isopropyl ester.

A solution of sodium methoxide (325mg, 6.1 mmol) and hydroxylamine hydrochloride (396mg, 6.1 mmol) in methanol (15mL) was stirred at room temperature for 2 hours. The solution was then filtered into a solution of 2S-[2R-(2,2-dimethyl-5-oxo-[1,3]-dioxolan-4S-yl)-4-methyl-pentanoylamino]-3-phenylpropionic acid isopropyl ester (800mg, 2.1mmol) in methanol (10mL). The reaction was allowed to stand at room temperature for 18 hours. The reaction mixture was concentrated under reduced pressure and the residue partitioned between ethyl acetate and water. The organic layer was washed with water, dried over magnesium sulphate, filtered and concentrated under reduced pressure. Recrystallisation from ethyl acetate gave 2S-[2R-(S-hydroxy-hydroxycarbamoyl-methyl)-4-methyl-pentanoylamine]-3-phenyl-propionic acid isopropyl ester as white crystalline material which was dried under vacuum (465mg, 58%).

### Preparative Example B

### 2S-[2R-(S-Hydroxycarbamoyl-methoxy-methyl)-4-methyl-pentanoylamino]-3-phenyl-propionic acid isopropyl ester.

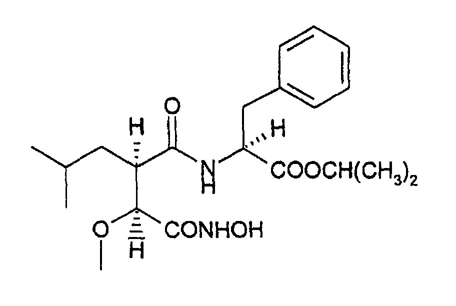

### (a) 2R-(S-Benzyloxycarbamoyl-methoxy-methyl)-4-methyl-pentanoic acid.

A solution of 3R-isobutyl-4S-methoxy-dihydrofuran-2,5-dione (WO 97/02239) (609mg, 3.27mmol), and O-benzylhydroxylamine (403mg, 3.27mmol) in ethyl acetate (5mL) was stirred at room temperature for 1 hour. The reaction mixture was concentrated under reduced pressure to provide 2R-(S-benzyloxycarbamoyl-methoxy-methyl)-4-methyl-pentanoic acid as a white foam (1.01g, 100%).

### (b) 2S-[2R-(S-Benzyloxycarbamoyl-methoxy-methyl)-4-methyl-pentanoylamino]-3-phenyl-propionic acid isopropyl ester.

A solution of 2R-(S-benzyloxycarbamoyl-methoxy-methyl)-4-methyl-pentanoic acid (1.01g, 3.3mmol) in tetrahydrofuran (15mL) at 0°C was treated with L-phenylalanine isopropyl ester (810mg, 3.9mmol) and N-(3-dimethylaminopropyl)-N'-ethylcarbodiimide hydrochloride (750mg, 3.9mmol). The reaction mixture was allowed to warm to ambient temperature and stirred for 18 hours. The solution was concentrated under reduced pressure and the residue taken up in DCM. This solution was washed with 1M hydrochloric acid, saturated sodium hydrogen carbonate and brine. The organic phase was dried with sodium sulphate, filtered and concentrated under reduced pressure. The product was purified by column chromatography, eluting with 2% methanol/DCM. Product-containing fractions were combined and concentrated under reduced pressure to provide 2S-[2R-(S-benzyloxycarbamoyl-methoxy-methyl)-4-methyl-pentanoylamino]-3-phenyl-propionic acid isopropyl ester as a white solid (1.39g, 85%).

### (c) 2S-[2R-(S-Hydroxycarbamoyl-methoxy-methyl)-4-methyl-pentanoylamino]-3-phenyl-propionic acid isopropyl ester.

A solution of 2S-[2R-(S-benzyloxycarbamoyl-methoxy-methyl)-4-methyl-pentanoylamino]-3-phenyl-propionic acid isopropyl ester (1.37g, 2.8mmol) in ethanol (30mL) was treated with palladium catalyst (274mg, 10%Pd/charcoal) as a slurry in ethyl acetate (5mL). Hydrogen gas was passed through the suspension for 2 hours. The reaction mixture was filtered and concentrated under reduced pressure. The product was recrystallised from ethyl acetate/hexane to provide 2S-[2R-(S-hydroxycarbamoyl-methoxy-methyl)-4-methyl-pentanoylamino]-3-phenyl-propionic acid isopropyl ester as a white solid (778mg, 70%).

### Preparative Example C

### 2S-(3S-Hydroxycarbamoyl-2R-isobutyl-hex-5-enoylamino)-3-phenylpropionic acid isopropyl ester.

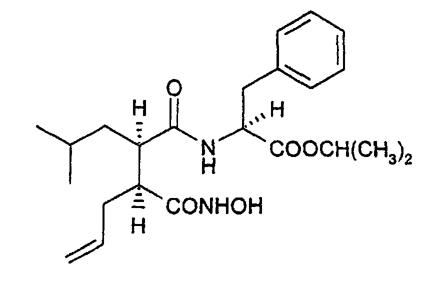

### (a) 2S-(3S-tert-Butoxycarbonyl-2R-isobutyl-hex-5-enoylamino)-3-phenylpropionic acid isopropyl ester.

A solution of L-phenylalanine isopropyl ester (3.9g, 18.8mmol) in DMF (15mL) was cooled in an ice-water bath and treated with 3S-tert-butoxycarbonyl-2R-isobutyl-hex-5-enoic acid pentafluorophenyl ester (9.03g, 20.7mmol). The reaction was allowed to warm to room temperature and stirred overnight. The reaction mixture was concentrated under reduced pressure. The residue was taken up in ethyl acetate and washed with 1M hydrochloric acid, 1M sodium carbonate and brine. The solution was dried over sodium sulphate, filtered and concentrated under reduced pressure. The product was purified by column chromatography using a gradient elution of 100% DCM to 10% methanol/DCM. Product containing fractions were combined and solvent removed to yield 2S-(3S-tert-butoxycarbonyl-2R-isobutyl-hex-5-enoylamino)-3-phenylpropionic acid isopropyl ester as a yellow solid (3.5g, 41%).

### (b) 2S-(3S-Hydroxycarbonyl-2R-isobutyl-hex-5-enoylamino)-3-phenylpropionic acid isopropyl ester

A solution of 2S-(3S-tert-butoxycarbonyl-2R-isobutyl-hex-5-enoylamino)-3-phenylpropionic acid isopropyl ester (3.5g, 7.6mmol) in a mixture of TFA and DCM (1:1, 10mL) was allowed to stand at 5°C overnight. The reaction mixture was concentrated under reduced pressure. Addition of ether to the residue gave 2S-(3S-hydroxycarbonyl-2R-isobutyl-hex-5-enoylamino)-3-phenylpropionic acid isopropyl ester as a white solid (261mg, 8%).

### (c) 2S-(3S-Hydroxycarbamoyl-2R-isobutyl-hex-5-enoylamino)-3-phenylpropionic acid isopropyl ester.

A solution of 2S-(3S-hydroxycarbonyl-2R-isobutyl-hex-5-enoylamino)-3-phenylpropionic acid isopropyl ester (260mg, 0.64mmol) in DMF (10mL) was cooled in an ice-water bath. N-(3-dimethylaminopropyl)-N'-ethylcarbodiimide hydrochloride (148mg, 0.77mmol) and HOBT (104mg, 0.77mmol) were added with stirring. The reaction was allowed to warm to room temperature and after 2 hours a solution of hydroxylamine hydrochloride (67mg, 0.96mmol) and NMM (0.1mL, 0.96mmol) in DMF (5mL) added. After stirring overnight the reaction mixture was concentrated under reduced pressure and the product was purified by chromatography on acid-washed silica using 5-10% methanol in DCM. Recrystallisation from from ethyl acetate/hexane provided 2S-(3S-hydroxycarbamoyl-2R-isobutyl-hex-5-enoylamino)-3-phenylpropionic acid isopropyl ester as a white solid (12mg, 4%).

### Preparative Example D

### 2S-(3S-Hydroxycarbamoyl-2R-isobutyl-hex-5-enoylamino)-2-phenylethanoic acid cyclopentyl ester.

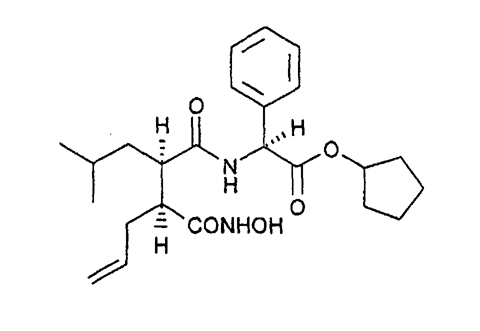

The title compound was prepared using an analogous route to that described for Preparative Example C using L-phenylglycine cyclopentyl ester in place of L-phenylalanine isopropyl ester.

### Example 1

### 2-[2R-(S-Hydroxy-hydroxycarbamoyl-methyl)-4-methyl-pentanoylamine]-2-phenyl-ethanoic acid cyclopentyl ester

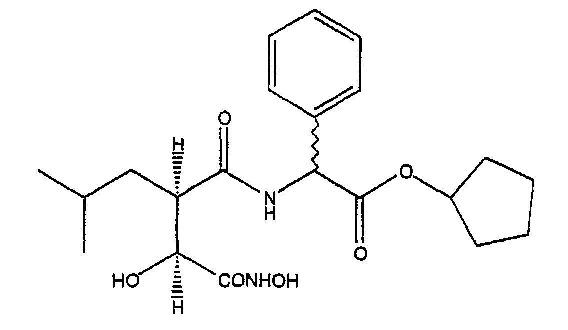

Prepared using procedures similar to those described in Preparative Example A using phenylglycine cyclopentyl ester.

### Diastereoisomer A

¹H-NMR; δ (MeOD), 7.4-7.29 (5H, m), 5.43 (1H, s), 5.2-5.14 (1H, m), 4.02 (1H, d, J=6.9Hz), 2.94-2.85 (1H, m), 1.91-1.34 (10H, bm), 1.25-1.14 (1H, m) and 0.86 (6H, dd, J=6.5, 11.5Hz).

¹³C-NMR; δ (MeOD), 175.8, 171.8, 171.4, 137.8, 129.8, 129.4, 128.6, 80.0, 73.2, 58.5, 49.2, 39.1, 33.3, 33.3, 26.8, 24.5, 24.4, 23.7 and 22.1.

### Diastereoisomer B

¹H-NMR; δ (MeOD), 7.33-7.19 (5H, m), 5.3 (1H, s), 5.11-5.06 (1H, m), 3.81 (1H, d, J=7.3Hz), 2.83-2.74 (1H, m), 1.83-1.45 (10H, bm), 1.12-1.03 (1H, m) and 0.88-0.81 (6H, dd, J=6.4, 12.3Hz). ¹³C-NMR; δ (MeOD), 175.8, 171.8, 171.5, 137.3, 129.8, 129.5, 128.8, 79.9, 73.3, 58.7, 48.9, 39.2, 33.3, 33.3, 26.7, 24.5, 24.5, 24.0 and 22.2.

### Example 2

### 2-(3S-Hydroxycarbamoyl-2R-isobutyl-hex-5-enoytamino)-2-phenyiethanoic acid isopropyl ester

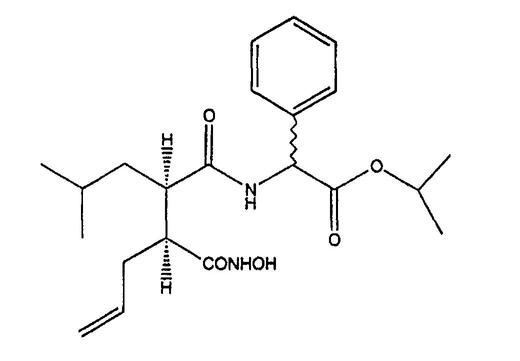

Prepared using methods similar to those described in Preparative Example D, using phenylglycine isopropyl ester.

### Diastereoisomer A

¹H-NMR; δ (MeOD), 7.34-7.24 (5H, m), 5.59-5.42 (1H, m), 5.36 (1H, s), 5.02-4.77 (3H, m), 2.63-2.53 (1H, m), 2.17-2.02 (2H, m), 1.89-1.78 (1H, m), 1.63-1.45 (2H, m), 1.18 (3H, d, J=6.3Hz), 1.05 (3H, d, J=6.2Hz), 1.00-0.93 (1H, m), 0.88 (3H, d, J=6.5Hz) and 0.81 (3H, d, J=6.5Hz). ¹³C-NMR; δ (MeOD), 176.2, 172.4, 171.3, 137.6, 136.0, 129.9, 129.6, 129.0, 117.4, 70.5, 58.7, 47.4, 41.5, 36.0, 26.7, 24.5, 21.9, 21.7 and 21.7.

### Diastereoisomer B

¹H-NMR; δ (MeOD), 7.4-7.34 (5H, m), 5.77-5.61 (1H, m), 5.42 (1H, s), 5.1-4.98 (3H, m), 2.7-2.6 (1H, m), 2.44-2.17 (3H, m), 1.61-1.5 (1H, m), 1.42-1.29 (1H, m), 1.25 (3H, d, J=6.3Hz), 1.13 (3H, d, J=6.2Hz), 1.09-1.00 (1H, m) and 0.81 (6H, d, J=6.4Hz). ¹³C-NMR; δ (MeOD), 176.4, 172.5, 171.5, 137.2, 136.4, 129.9, 129.6, 129.0, 117.5, 70.5, 58.8, 48.4, 47.4, 41.3, 36.0, 27.1, 24.3, 21.9, 21.8 and 21.6.

### Example 3

### 2-[2R-(S-Hydroxycarbamoyl-methoxy-methyl)-4-methyl-pentanoylamino]-3-pnenylethanoic acid cyclopentyl ester

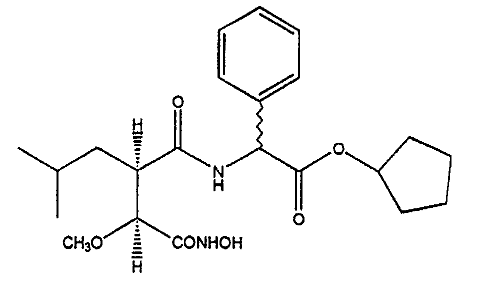

Prepared using methods similar to those described in Preparative Example B, using phenylglycine cyclopentyl ester.

### Diastereoisomer A

¹H-NMR; δ (MeOD), 8.83 (1H, d, J=6.6Hz), 7.48-7.29 (5H, m), 5.44-5.42 (1H, m), 5.20-5.16 (1H, m), 3.53 (1H, d, J=9.7Hz), 3.17 (3H, s), 2.89-2.79 (1H, m), 1.90-1.54 (10H, bm), 1.06-0.99 (1H, m), 0.95 (3H, d, J=6.5Hz) and 0.90 (3H, d, J=6.4Hz). ¹³C-NMR; δ (MeOD), 175.3, 171.6, 169.4, 137.5, 129.7, 129.4, 128.7, 83.1, 79.9, 58.7, 58.1, 48.5, 38.4, 33.4, 33.3, 26.7, 24.6, 24.5, 24.3 and 21.8.

### Diastereoisomer B

¹H-NMR; δ (MeOD), 7.39-7.30 (5H, m), 5.45 (1H, s), 5.21-5.15 (1H, m), 3.59 (1H, d, J=9.4Hz), 3.29 (3H, s), 2.89-2.79 (1H, m), 1.93-1.49 (9H, bm), 1.42-1.21 (1H, m), 1.01 (1H, ddd, J=3.7, 9.9, 13.3Hz), 0.83 (3H, d, J=6.5Hz) and 0.79 (3H, d, J=6.6Hz). ¹³C-NMR; δ (MeOD), 175.1, 171.5, 169.5, 137.9, 129.7, 129.4, 128.7, 83.0, 79.8, 58.5, 58.3, 48.6, 38.5, 33.3, 27.8, 24.5, 24.4, 24.1 and 21.7.

### Example 4

### 2-(3S-Hydroxycarbamoyl-2R-isobutyl-hex-5-enoylamino)-2-(4-methoxyphenyl)ethanoic acid cyclopentyl ester

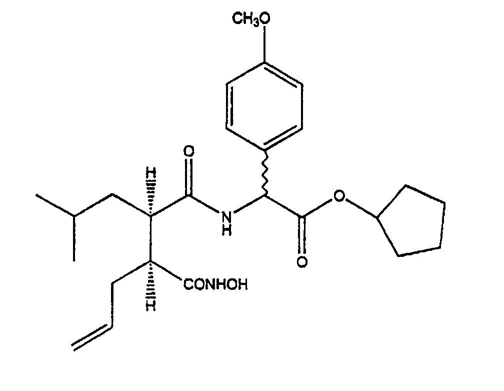

Prepared using methods similar to those described in Preparative Example D, using 4-methoxyphenylglycine cyclopentyl ester.

### Diastereoisomer A

¹H-NMR; δ (MeOD), 8.94 (1H, d, J=6.4Hz), 7.32 (2H, d, J=8.7Hz), 6.93 (2H, d, J=8.7Hz), 5.67-5.50 (1H, m), 5.36-5.33 (1H, m), 5.20-5.14 (1H, m), 4.93-4.87 (2H, m), 3.79 (3H, s), 2.68-2.59 (1H, m), 2.24-2.09 (2H, m), 1.97-1.55 (11H, bm), 1.11-1.00 (1H, m), 0.95 (3H, d, J=6.5Hz) and 0.88 (3H, d, J=6.5Hz). ¹³C-NMR; δ (MeOD), 176.2, 172.4, 171.9, 161.4, 136.0, 130.2, 129.4, 117.4, 115.2, 79.7, 58.2, 55.8, 48.3, 47.3, 41.5, 36.0, 33.4, 33.3, 26.7, 24.6, 24.5 and 21.7.

### Diastereoisomer B

¹H-NMR; δ (MeOD), 8.96 (1H, d, J=6.7Hz), 7.29 (2H, d, J=8.7Hz), 6.93 (2H, d, J=8.7Hz), 5.77-5.61 (1H, m), 5.32 (1H, s), 5.20-5.15 (1H, m), 5.09-4.97 (2H, m), 3.80 (3H, s), 2.64 (1H, dt, J=3.3, 11.4, 13.5Hz), 2.43-2.16 (3H, m), 1.91-1.49 (9H, bm), 1.42-1.29 (1H, m), 1.05 (1H, ddd, J=3.3, 10.1, 13.2Hz) and 0.81 (6H, d, J=6.5Hz). ¹³C-NMR; δ (MeOD), 176.3, 172.5, 172.0, 161.4, 136.4, 130.2, 129.0, 117.5, 115.2, 79.8, 58.2, 55.8, 48.4, 47.4, 41.3, 36.1, 33.4, 27.1, 24.5, 24.3 and 21.6.

### Example 5

### 2-(3S-Hydroxycarbamoyl-2R-isobutyl-hex-5-enoylamino)-2-(thien-2-yl)ethanoic acid cyclopentyl ester

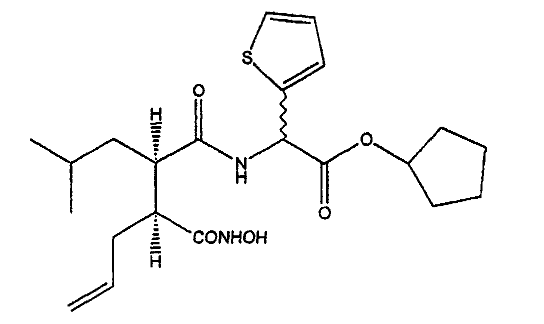

Prepared using methods similar to those described in Preparative Example D, using thien-2-ylglycine cyclopentyl ester.

### Diastereoisomer A

¹H-NMR; δ (MeOD), 7.41 (1H, dd, J=5.1, 1.2Hz), 7.12 (1H, d, J=3.5Hz), 7.01 (1H, dd, J=5.1, 3.5Hz), 5.72 (1H, s), 5.69-5.52 (1H, m), 5.26-5.18 (1H, m), 5.00-4.89 (2H, m), 2.70-2.59 (1H, m), 2.28-2.13 (2H, m), 2.09-1.50 (11H, m), 1.05 (1H, ddd, J=13.8, 11.0, 2.9Hz), 0.93 (3H, d, J=6.4Hz) and 0.87 (3H, d, J=6.5Hz). ¹³C-NMR; δ (MeOD), 176.5, 172.7, 171.1, 139.5, 136.4, 128.4, 128.3, 127.7, 117.9, 80.7, 54.1, 48.7, 47.7, 41.9, 36.5, 33.8, 33.7, 27.2, 25.1, 25.0, 24.9, and 22.1.

### Diastereoisomer B

¹H-NMR; δ (MeOD), 7.42 (1H, dd, J=5.0, 0.7Hz), 7.10 (1H, d, J=3.6Hz), 7.01 (1H, dd, J=5.0, 3.6Hz), 5.79-5.59 (2H, m), 5.28-5.19 (1H, m), 5.10-4.94 (2H, m), 2.71-2.59 (1H, m), 2.36-2.16 (3H, m), 1.97-1.34 (10H, m), 1.13-1.00 (1H, m), 0.86 (3H, d, J=6.2Hz) and 0.84 (3H, d, J=6.3Hz). ¹³C-NMR; δ (MeOD), 176.7, 172.8, 171.2, 139.3, 136.7, 128.3, 128.2, 127.6, 117.9, 80.7, 54.2, 48.8, 47.8, 41.7, 36.4, 33.8, 27.5, 25.1, 25.0, 24.8 and 22.1.

### Example 6

### 2-(3S-Hydroxycarbamoyl-2R-isobutyl-hex-5-enoylamino)-2-(thien-3-yl)ethanoic acid cyclopentyl ester

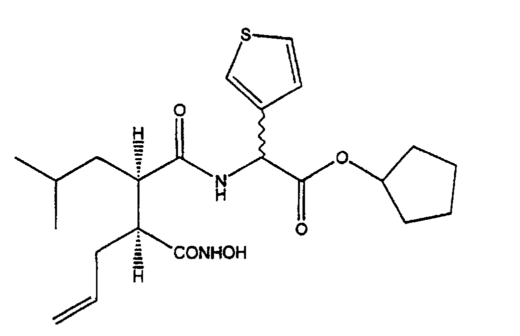

Prepared using methods similar to those described in Preparative Example D, using thien-3-ylglycine cyclopentyl ester.

### Diastereoisomer A

¹H-NMR; δ (MeOD), 7.48-7.42 (2H, m), 7.13 (1H, dd, J=4.2, 2.0Hz), 5.69-5.52 (2H, m), 5.21-5.16 (1H, m), 4.98-4.90 (2H, m), 2.71-2.59 (1H, m), 2.28-2.11 (2H, m), 2.00-1.50 (11H, m), 1.12-0.98 (1H, m), 0.94 (3H, d, J=6.4Hz) and 0.88 (3H, d, J=6.5Hz). ¹³C-NMR; δ (MeOD), 176.6, 172.8, 171.8, 137.8, 136.4, 128.3, 128.0, 125.2, 117.9, 80.3, 54.6, 41.9, 36.5, 33.8, 33.8, 27.1, 25.0, 24.9 and 22.1.

### Diastereoisomer B

¹H-NMR; δ (MeOD), 7.45 (1H, dd, J=4.9, 3.0Hz), 7.43-7.40 (1H, m), 7.12 (1H, dd, J=5.0, 1.3Hz), 5.68 (1H, ddt, J=17.0, 10.1, 6.8Hz), 5.53 (1H, s), 5.23-5.17 (1H, m), 5.10-4.96 (2H, m), 2.70-2.60 (1H, m), 2.41-2.16 (3H, m), 1.94-1.49 (9H, m), 1.44-1.29 (1H, m), 1.05 (1H, ddd, J=12.9, 10.3, 3.3Hz), 0.84 (3H, d, J=6.5Hz) and 0.83 (3H, d, J=6.5Hz).

### Biological Example

The compounds of examples 1-6 were tested in the following cell proliferation assay, to determine their respective capacities to inhibit proliferation of the cell type in question.

A human cell line, namely a histiocytic lymphoma (U937), was seeded into 30mm² tissue culture wells, in the appropriate culture medium supplemented with 10% fetal calf serum at a density of 250 cells/mm². Six hours later the test compounds were added in the same culture medium to the cells to give a final concentration of 6µM. Control wells contained cells supplemented with the same culture medium containing the equivalent amount of drug vehicle, which in this case was DMSO at a final concentration of 0.08%. After 72 hours in culture the cells were pulsed for 3 hours with [methyl-³[H] Thymidine] (2µCi/ml) and then harvested onto filter mats and DNA associated radioactivity counted. Results were expressed as percentage of control ³[H] Thymidine incorporation (n=6± 1 stdv). Inhibition of proliferation was observed with all test compounds.

## Claims

1. A compound selected from the group consisting of:
2(R or S)-[2R-(S-Hydroxy-hydroxycarbamoyl-methyl)-4-methyl-pentanoylamine]-2-phenyl-ethanoic acid cyclopentyl ester
2(R or S)-(3S-Hydroxycarbamoyl-2R-isobutyl-hex-5-enoylamino)-2-phenylethanoic acid isopropyl ester
2(R or S)-[2R-(S-Hydroxycarbamoyl-methoxy-methyl)-4-methyl-pentanoylamino]-3-phenylethanoic acid cyclopentyl ester
2(R or S)-(3S-Hydroxycarbamoyl-2R-isobutyl-hex-5-enoylamino)-2-(4-methoxyphenyl)ethanoic acid cyclopentyl ester
2(R or S)-(3S-Hydroxycarbamoyl-2R-isobutyl-hex-5-enoylamino)-2-(thien-2-yl)ethanoic acid cyclopentyl ester
2(R or S)-(3S-Hydroxycarbamoyl-2R-isobutyl-hex-5-enoylamino)-2-(thien-3-yl)ethanoic acid cyclopentyl ester,
and pharmaceutically or veterinarily acceptable salts, hydrates or solvates thereof.

2. A compound as claimed in claim 1 which is the 2-S diastereomer.

3. A pharmaceutical or veterinary composition comprising a compound as claimed in claim 1 or claim 2, together with a pharmaceutically or veterinarily acceptable excipient or carrier.

4. The use of a compound compound as claimed in claim 1 or claim 2 in the manufacture of a composition for inhibiting proliferation of tumour cells in mammals.

5. The use as claimed in claim 4 wherein the cell proliferation treated is over-proliferation of lymphoma, leukemia, myeloma, adenocarcinoma, carcinoma, mesothelioma, teratocarcinoma, choriocarcinoma, small cell carcinoma, large cell carcinoma, melanoma, retinoblastoma, fibrosarcoma, leiomyosarcoma, glioblastoma or endothelioma cells.

## Patentansprüche

1. Verbindung, ausgewählt aus der Gruppe, bestehend aus:
2(R oder S)-[2R-(S-Hydroxy-hydroxycarbamoyl-methyl)-4-methyl-pentanoylamin]-2-phenyl-ethansäurecyclopentylester
2(R oder S)-(3S-Hydroxycarbamoyl-2R-isobutyl-hex-5-enoylamino)-2-phenylethansäureisopropylester
2(R oder S)-[2R-(S-Hydroxycarbamoyl-methoxy-methyl)-4-methyl-pentanoylamino]-3-phenylethansäurecyclopentylester
2(R oder S)-(3S-Hydroxycarbamoyl-2R-isobutyl-hex-5-enoylamino)-2-(4-methoxyphenyl)ethansäurecyclopentylester
2(R oder S)-(3S-Hydroxycarbamoyl-2R-isobutyl-hex-5-enoylamino)-2-(thien-2-yl)ethansäurecyclopentylester
2(R oder S)-(3S-Hydroxycarbamoyl-2R-isobutyl-hex-5-enoylamino)-2-(thien-3-yl)ethansäurecyclopentylester
und pharmazeutisch oder veterinärmedizinisch annehmbare Salze, Hydrate oder Solvate davon.

2. Verbindung gemäss Anspruch 1, die das 2-S-Diastereomer ist.

3. Pharmazeutische oder veterinärmedizinische Zusammensetzung, umfassend eine Verbindung gemäss Anspruch 1 oder Anspruch 2 zusammen mit einem pharmazeutisch oder veterinärmedizinisch annehmbaren Exzipienten oder Träger.

4. Verwendung einer Verbindung gemäss Anspruch 1 oder 2 bei der Herstellung einer Zusammensetzung zur Inhibierung der Proliferation von Tumorzellen in Säugern.

5. Verwendung gemäss Anspruch 4, worin die behandelte Zellproliferation übermässige Proliferation von Lymphom-, Leukämie-, Myelom-, Adenokarzinom-, Karzinom-, Mesotheliom-, Teratokarzinom-, Chorionepitheliom-, Kleinzellkarzinom-, Grosszellkarzinom-, Melanom-, Retinoblastom-, Fibrosarkom-, Leiomyosarkom-, Glioblastom- oder Endotheliomzellen ist.

## Revendications

1. Composé choisi dans le groupe constitué par :
- un cyclopentylester de l'acide 2(R ou S)-[2R-(S-hydroxy-hydroxycarbamoyl-méthyl)-4-méthyl-pentanoylamine]-2-phényl-éthanoïque,
- un isopropylester de l'acide 2(R ou S)-(3S-hydroxy-carbamoyl-2R-isobutyl-hex-5-ènoylamino)-2-phényl-éthanoïque,
- un cyclopentylester de l'acide 2(R ou S)-[2R-(S-hydroxycarbamoyl-méthoxy-méthyl)-4-méthyl-pentanoylamino]-3-phényl-éthanoïque,
- un cyclopentylester de l'acide 2(R ou S)-(3S-hydroxycarbamoyl-2R-isobutyl-hex-5-ènoylamino)-2-(4-méthoxyphényl)-éthanoïque,
- un cyclopentylester de l'acide 2(R ou S)-(3S-hydroxycarbamoyl-2R-isobutyl-hex-5-ènoylamino)-2-(thién-2-yl)-éthanoïque,
- un cyclopentylester de l'acide 2(R ou S)-(3S-hydroxycarbamoyl-2R-isobutyl-hex-5-ènoylamino)-2-(thién-3-yl)-éthanoïque,
et leurs sels, hydrates ou solvates acceptables d'un point de vue pharmaceutique ou vétérinaire.

2. Composé selon la revendication 1, qui est le 2-S diastéréomère.

3. Composition pharmaceutique ou vétérinaire comprenant un composé selon la revendication 1 ou 2, avec un excipient ou un véhicule acceptable d'un point de vue pharmaceutique ou vétérinaire.

4. Utilisation d'un composé selon la revendication 1 ou 2 dans la préparation d'une composition destinée à inhiber la prolifération des cellules tumorales chez les mammifères.

5. Utilisation selon la revendication 4, dans laquelle la prolifération cellulaire traitée est une sur-prolifération cellulaire de types lymphomes, leucémies, myélomes, adénocarcinomes, carcinomes, mésothéliomes, tératocarcinomes, chorio-carcinomes, carcinomes à petites cellules, carcinomes à grandes cellules, mélanomes, rétinoblastomes, fibrosarcomes, léiomyosarcomes, glioblastomes ou endothéliomes.
